# EUROPÄISCHE PATENTANMELDUNG

(11) **EP 4 756 424 A1**
(43) Veröffentlichungstag der Anmeldung: **10.06.2026**
(21) Anmeldenummer: 25218365.2
(22) Anmeldetag: 25.11.2025
(51) Int. Cl.: G01N 33/00, B60L 3/00, H01M 10/48

(54) **SENSOR UND SENSOR-STEUERGERÄT ZUR ÜBERWACHUNG EINER MESSGRÖSSE**

(30) Priorität: 06.12.2024 DE 102024136563
(71) Anmelder: Volkswagen Aktiengesellschaft, 38440 Wolfsburg (DE)
(72) Erfinder: Voss, Fabian, 38440 Wolfsburg (DE)

(57) **Zusammenfassung**

Die Erfindung betrifft einen Sensor (2) zur Überwachung einer Messgröße, wobei der Sensor (2) einen Versorgungsspannungseingang (5), einen Sensorausgang(8) und einen Systemzustandseingang (7) aufweist, wobei der Sensor (3) derart ausgebildet ist, bei einem eingeschalteten System am Sensorausgang (8) ein erstes Signal mit einem ersten konstanten Pegel (P1) zu erzeugen, wobei bei Erfassung einer Messgröße größer einem Schwellwert mindestens ein zweites Signal mit mindestens einem zweiten Pegel (P2) erzeugt wird, wobei bei einem ausgeschaltetem System der Sensor (2) sich in einen Schlafmodus schaltet und kein Signal am Sensorausgang (8) erzeugt und sich periodisch aufweckt und die Messgröße erfasst, wobei im aufgeweckten Zustand mindestens ein erstes Signal mit dem ersten Pegel (P1) am Sensorausgang (8) anliegt, sowie eine Anordnung (1) mit einem Sensor (2) und einem Steuergerät (3).

## Beschreibung

Die Erfindung betrifft einen Sensor und eine Anordnung mit einem solchen Sensor.

Sensoren sind für die verschiedensten Anwendungen bekannt. Sensoren brauchen häufig eine Versorgungsspannung, sodass diese einen entsprechenden Ruhestrombedarf haben. Daher ist es bekannt, diese auszuschalten, wenn das System, in dem diese angeordnet sind, ausgeschaltet ist. Verschärft wird dieses Problem bei Sensoren, die permanent ein Ausgangssignal erzeugen müssen, um so ihre Funktionsfähigkeit zu signalisieren. Andererseits existieren Systeme, die Komponenten aufweisen, die auch im ausgeschalteten Zustand des Systems in einen kritischen Zustand kommen könnten.

Der Erfindung liegt das technische Problem zugrunde, einen Sensor sowie eine Anordnung mit einem solchen Sensor zu schaffen, die das zuvor beschriebene Problem zumindest reduzieren.

Die Lösung des technischen Problems ergibt sich durch einen Sensor mit den Merkmalen des Anspruchs 1 sowie eine Anordnung mit den Merkmalen des Anspruchs 4. Weitere vorteilhafte Ausgestaltungen der Erfindung ergeben sich aus den Unteransprüchen.

Der Sensor zur Überwachung einer Messgröße weist einen Versorgungsspannungseingang, einen Sensorausgang und einen Systemzustandseingang auf. Der Sensor ist derart ausgebildet, bei einem eingeschalteten System am Sensorausgang ein erstes Signal mit einem ersten konstanten Pegel zu erzeugen. Dieses erste Signal wird erzeugt, auch wenn keine Messgröße erfasst wird und signalisiert die Funktionsfähigkeit des Sensors, die dann beispielsweise von einem Steuergerät überwacht wird. Weiter ist der Sensor derart ausgebildet, dass bei Erfassung einer Messgröße größer einem Schwellwert mindestens ein zweites Signal mit mindestens einem zweiten Pegel erzeugt wird. Der zweite Pegel ist dabei größer als der erste Pegel. Die Pegel können dabei Analogwerte sein (z.B. Spannungswerte) oder Digitalwerte sein (z.B. PWM-Signale oder Binärwerte). Weiter ist der Sensor derart ausgebildet, dass sich dieser bei einem ausgeschalteten System in einen Schlafmodus schaltet und kein Signal am Sensorausgang erzeugt. Entsprechend benötigt der Sensor in diesem Schlafmodus keinen oder einen minimalen Ruhestandsbedarf. Der Sensor ist weiter derart ausgebildet, sich periodisch aufzuwecken, wobei im aufgeweckten Zustand die Messgröße erfasst wird und mindestens ein erstes Signal mit dem ersten Pegel anliegt. Wird dann keine Messgröße größer dem Schwellwert erfasst, geht der Sensor wieder in den Schlafmodus. Hierdurch kann der Sensor quasi permanent eine Komponente überwachen, wobei der Ruhestrombedarf durch den Schlafmodus reduziert wird. Dabei kann die Aufwachzeit gleich oder kürzer als die Schlafzeit sein, so lange nur sichergestellt ist, dass eine Messung der Messgröße durchgeführt werden kann.

In einer Ausführungsform ist der Sensor als Gassensor ausgebildet, der beispielsweise eine Gaskonzentration einer Batteriezelle bzw. Batterieeinheit überwacht.

Weiter vorzugsweise ist der Sensor als analoger Sensor ausgebildet, d.h. das mindestens die Pegel am Sensorausgang Analog-Signale sind. Der Vorteil von Analog-Sensoren ist, dass häufig eine Anpassung von Auslöseschwellen einfacher angepasst werden kann, was später noch näher erläutert wird.

Die Frequenz, mit der der Sensor periodisch aufgeweckt wird, ist dann abhängig, wie schnell sich der Zustand einer zu überwachenden Komponente ändern kann. Bei einem Gassensor zur Überwachung von Batteriezellen bzw. Batterieeinheiten ist die Frequenz vorzugsweise 0,01 - 1 Hz, weiter vorzugsweise 0,1 - 0,5 Hz.

Die Anordnung umfasst ein Steuergerät und einen zuvor beschriebenen Sensor, wobei der Sensorausgang mit dem Steuergerät verbunden ist. Das Steuergerät ist derart ausgebildet, bei einem eingeschalteten System und einem Signal unterhalb des ersten Pegels auf einen defekten Sensor zu schließen. Das Steuergerät kann dann beispielsweise eine Warnmeldung erzeugen und/oder andere Gegenmaßnahmen einleiten.

In einer weiteren Ausführungsform ist das Steuergerät derart ausgebildet, bei einem eingeschalteten System und einem Signal oberhalb eines dritten Pegels, wobei der dritte Pegel größer als der zweite Pegel ist, auf einen defekten Sensor zu schließen, beispielsweise weil ein Kurzschluss gegen die Versorgungsspannung vorliegt. Der erste Pegel und der dritte Pegel definieren also einen zulässigen Wertebereich am Sensorausgang.

In einer weiteren Ausführungsform ist das Steuergerät derart ausgebildet, bei einem ausgeschalteten System in einem Sleep-Modus zu sein und durch ein Signal größer dem zweiten Pegel aufzuwachen.

Vorzugsweise ist das Steuergerät weiter derart ausgebildet, im aufgeweckten Zustand den Systemzustandseingang des Sensors auf "eingeschaltetes System" zu wechseln bzw. zu schalten.

In einer weiteren Ausführungsform ist das Steuergerät derart ausgebildet, im eingeschalteten Zustand und einem Signal größer dem zweiten Pegel (und kleiner dem dritten Pegel) auf einen Zustand der Komponente zu schließen, beispielsweise dass eine Batteriezelle defekt ist und ein Thermal Runaway einer Batterieeinheit droht.

In einer weiteren Ausführungsform ist das Steuergerät derart ausgebildet, die Pegel in Abhängigkeit eines Steuersignals und/oder erfasster Messwerte anzupassen. So kann beispielsweise das Steuergerät auf eine festgestellte Drift der Messwerte bzw. der Signale über die Lebensdauer des Sensors reagieren und gegebenenfalls gehäufte Falschwarnungen vermeiden.

In einer weiteren Ausführungsform ist die Komponente eine Batterieeinheit und/oder das Steuergerät ein Batterie-Management-Steuergerät.

Die Erfindung wird nachfolgend anhand eines bevorzugten Ausführungsbeispiels näher erläutert. Die Figuren zeigen:
- Fig. 1: eine schematische Darstellung einer Anordnung mit einem Sensor und einem Steuergerät,
- Fig. 2: eine schematische Darstellung eines Signals an einem Sensorausgang über der Zeit bei einem eingeschalteten System und
- Fig. 3: eine schematische Darstellung eines Signals an einem Sensorausgang über der Zeit bei einem ausgeschalteten System.

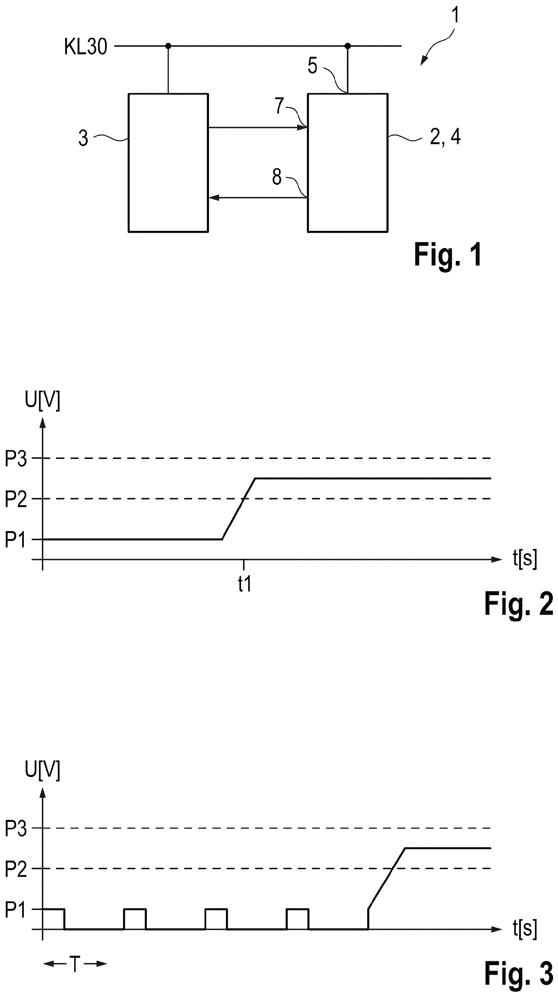

In der Fig. 1 ist schematisch eine Anordnung 1 dargestellt, die einen Sensor 2 und ein Steuergerät 3 aufweist. Der Sensor 2 ist vorzugsweise ein Gassensor 4. Die Anordnung 1 ist beispielsweise Bestandteil eines Batteriesystems eines Traktionsnetzes eines Elektrofahrzeugs, wobei der Gassensor 4 beispielsweise die CO- oder H₂-Konzentration einer Batterieeinheit überwacht, um bei Defekt einer Batteriezelle Gegenmaßnahmen zur Vermeidung eines Thermal Runaways der anderen Batteriezellen einzuleiten und Warnmeldungen zu erzeugen. Der Sensor 2 weist einen Versorgungsspannungseingang 5 auf, der an eine Dauerspannung KL30 angeschlossen ist. Ebenso kann das Steuergerät 3 einen Spannungsversorgungseingang 6 aufweisen, an der die Dauerspannung KL30 anliegt. Die Spannung kann dabei von einer Niedervoltbatterie und/oder über einen DC/DC-Wandler aus der zu überwachenden Batterieeinheit zur Verfügung gestellt werden.

Weiter weist der Sensor 2 einen Systemzustandseingang 7 auf, der mit dem Steuergerät 3 verbunden ist, sowie einen Sensorausgang 8, der ebenfalls mit dem Steuergerät 3 verbunden ist. Der Systemzustandseingang 7 signalisiert dem Sensor 2, ob das Batteriesystem eingeschaltet ist oder nicht, insbesondere ob das Steuergerät 3 eingeschaltet ist oder nicht. Ist das Steuergerät 3 eingeschaltet, so liegt an dem Systemzustandseingang 7 ein Signal an. Ist der Sensor 2 ein digitaler Sensor, so liegt beispielsweise eine logische 1 an. Ist der Sensor 2 ein analoger Sensor, so liegt beispielsweise eine Spannung größer einem Schwellwert an. Der Schwellwert beträgt beispielsweise 6 V.

Anhand der Fig. 2 wird nun zunächst die Funktionsweise des Sensors 2 bei eingeschaltetem System erläutert. Der Sensor erzeugt an seinem Sensorausgang ein erstes Spannungssignal U mit einem ersten konstanten Pegel P1, auch wenn keine Messgröße erfasst wird (also kein CO oder H₂) bzw. die Messgröße unterhalb einer Erfassungsschwelle ist. Dieses Signal am Sensorausgang 8 wird vom Steuergerät 3 gelesen. Solange dieses Signal mit dem Pegel P1 erfasst wird, weiß das Steuergerät 3, dass der Sensor 2 aktiv ist und nicht defekt ist. Sinkt hingegen das Signal unter den ersten Pegel P1 oder überschreitet einen dritten Pegel P3, so schließt das Steuergerät 3 auf einen defekten Sensor 2 und führt Gegenmaßnahmen aus. Tritt dann beispielsweise Gas aus einer defekten Batteriezelle der Batterieeinheit aus, so steigt das Signal am Sensorausgang 8, wobei zu einem Zeitpunkt t₁ ein zweiter Pegel P2 bzw. Schwellwert überschritten wird, was das Steuergerät 3 als einen potentiellen bevorstehenden Thermal Runaway der Batterieeinheit erfasst und entsprechende Maßnahmen ergreift.

Wird nun das System ausgeschaltet, so gehen das Steuergerät 3 und der Sensor 2 in einen Schlafmodus, wobei andere Komponenten des Systems ganz ausgeschaltet werden können. An dem Systemzustandseingang 7 liegt dann beispielsweise eine Spannung unter dem Schwellwert an (z.B. kleiner 6 V). Der Sensor 2 ist derart ausgebildet, dass sich dieser periodisch selbst aufweckt und eine Messung der Messgröße (z.B. der CO- oder H₂-Konzentration) vornimmt. Dabei liegt in den Phasen, wo der Sensor 2 aufgeweckt ist, wieder eine Spannung mit einem Pegel P1 am Sensorausgang 8 an. Dabei ist die Phase, wo der Sensor 2 aufgeweckt ist, kürzer als die Phase, wo der Sensor 2 im Schlafmodus ist und die Spannung am Sensorausgang 7 null ist. Dies ist in Fig. 3 dargestellt. Dabei ist die Periodendauer T der Kehrwert der Frequenz, mit der der Sensor 2 periodisch aufgeweckt wird. Erfasst dann der Sensor 2 eine Messgröße größer einem Schwellwert, so erzeugt dieser am Sensorausgang 8 ein Signal größer dem zweiten Pegel P2. Dieses Signal größer dem zweiten Pegel P2 ist ein Aufwecksignal für das Steuergerät 3. Das Steuergerät 3 erzeugt ein Signal am Systemzustandseingang 7, dass das System eingeschaltet ist. Des Weiteren führt das Steuergerät 3 Maßnahmen durch, beispielsweise erzeugt eine Warnmeldung oder initiiert weitere Messungen, um die Gefahr eines Thermal Runaway zu verifizieren. Durch den Sleep-Modus mit periodischer Selbstaufweckung überwacht der Sensor 2 quasi permanent die Komponente, wobei der Ruhestrombedarf signifikant reduziert wird.

### Bezugszeichenliste

- 1: Anordnung
- 2: Sensor
- 3: Steuergerät
- 4: Gassensor
- 5: Versorgungsspannungseingang
- 6: Spannungsversorgungseingang
- 7: Systemzustandseingang
- 8: Sensorausgang
- P1: erster Pegel
- P2: zweiter Pegel
- P3: dritter Pegel
- T: Periodendauer

## Patentansprüche

1. Sensor (2) zur Überwachung einer Messgröße, wobei der Sensor (2) einen Versorgungsspannungseingang (5), einen Sensorausgang(8) und einen Systemzustandseingang (7) aufweist, wobei der Sensor (3) derart ausgebildet ist, bei einem eingeschalteten System am Sensorausgang (8) ein erstes Signal mit einem ersten konstanten Pegel (P1) zu erzeugen, wobei bei Erfassung einer Messgröße größer einem Schwellwert mindestens ein zweites Signal mit mindestens einem zweiten Pegel (P2) erzeugt wird, wobei bei einem ausgeschaltetem System der Sensor (2) sich in einen Schlafmodus schaltet und kein Signal am Sensorausgang (8) erzeugt und sich periodisch aufweckt und die Messgröße erfasst, wobei im aufgeweckten Zustand mindestens ein erstes Signal mit dem ersten Pegel (P1) am Sensorausgang (8) anliegt.

2. Sensor nach Anspruch 1, **dadurch gekennzeichnet, dass** der Sensor (2) als Gassensor (4) ausgebildet ist.

3. Sensor nach Anspruch 1 oder 2, **dadurch gekennzeichnet, dass** der Sensor (2) als analoger Sensor ausgebildet ist.

4. Anordnung (1), umfassend ein Steuergerät (3) und einen Sensor (2) nach einem der Ansprüche 1 bis 3, **dadurch gekennzeichnet, dass** der Sensorausgang (8) des Sensors (2) mit dem Steuergerät (3) verbunden ist, wobei das Steuergerät (3) derart ausgebildet ist, bei einem eingeschalteten System und einem Signal unterhalb des ersten Pegels (P1) auf einen defekten Sensor (2) zu schließen.

5. Anordnung nach Anspruch 4, **dadurch gekennzeichnet, dass** das Steuergerät (3) derart ausgebildet ist, bei einem eingeschalteten System und einem Signal oberhalb eines dritten Pegels (P3) größer als der zweite Pegel (P2) ist, auf einen defekten Sensor zu schließen.

6. Anordnung nach Anspruch 4 oder5, **dadurch gekennzeichnet, dass** das Steuergerät (3) derart ausgebildet ist, bei einem ausgeschalteten System in einem Sleep-Modus zu sein und durch ein Signal größer dem zweiten Pegel (P2) am Sensorausgang (8) aufzuwachen.

7. Anordnung nach Anspruch 6, **dadurch gekennzeichnet, dass** das Steuergerät (3) derart ausgebildet ist, im aufgewachten Zustand den Systemzustandseingang (7) auf eingeschaltetes System zu schalten.

8. Anordnung nach einem der Ansprüche 4 bis 7, **dadurch gekennzeichnet, dass** das Steuergerät (3) derart ausgebildet ist, im eingeschalteten Zustand und einem Signal größer dem zweiten Pegel (P2) auf einen Zustand einer Komponente zu schließen.

9. Anordnung nach einem der Ansprüche 4 bis 8, **dadurch gekennzeichnet, dass** das Steuersignal (3) derart ausgebildet ist, die Pegel (P1-P3) in Abhängigkeit eines Steuersignals und/oder erfasster Messwerte anzupassen.

10. Anordnung nach Anspruch 8 oder 9, **dadurch gekennzeichnet, dass** die Komponente eine Batterieeinheit und/oder das Steuergerät (3) ein Batterie-Management-Steuergerät ist.
